# EUROPEAN PATENT SPECIFICATION

(11) **EP 3 348 191 B1**
(45) Date of publication and mention of the grant of the patent: **23.02.2022**
(21) Application number: 18020067.7
(22) Date of filing: 10.12.2015
(51) Int. Cl.: A61B 5/08, A61B 5/083, A61B 5/097, G01N 33/497

(54) **CAPNOMETER**
KAPNOMETER
CAPNOMÈTRE

(30) Priority: 10.12.2014 GB 201421961
(43) Date of publication of application: 18.07.2018
(62) Divisional of application: 15823521.8
(73) Proprietor: Cambridge Respiratory Innovations Ltd, Cambridge, Cambridgeshire CB23 8BS (GB); Wideblue Ltd, Glasgow G20 0SP (GB); Gas Sensing Solutions Ltd, Cumbernauld, Glasgow G68 9HQ (GB)
(72) Inventor: CARTER, Julian, Dry Dayton, Cambridgeshire CB23 8BS (GB); WALSH, Jeremy, Dry Dayton, Cambridgeshire CB23 8BS (GB); PATEL, Nalinkumar, Cambridge, Cambridgeshire CB24 9HS (GB); MCGREGOR, Calum, Cumbernauld, Glasgow G68 9HQ (GB); GIBSON, Des, Cumbernauld, Glasgow G68 9HQ (GB); WADELL, Ewan, Cumbernauld, Glasgow G68 9HQ (GB); OVEREND, Russell, Glasgow G20 0SP (GB)
(74) Representative: Lees, Kate Jane

(56) References cited:
- EP-A2- 0 729 727
- US-A- 5 261 415
- US-A1- 2003 134 427
- US-A1- 2007 173 731

## Description

### FIELD OF THE INVENTION

This invention generally relates to a capnometer, in particular a capnometer utilising a reflecting geometry, and methods for determining a CO₂ level in a gas using a capnometer.

### BACKGROUND TO THE INVENTION

Non-dispersive infrared (IR) techniques utilise the 4.26 µm absorption band of CO₂ and are widely used in the gas analyser industry. Single collimated emission and detection at the 4.26 µm absorption band have been disclosed in, e.g. US 4,423,739, US 4,200,791, US 4,013,260 and US 3,893,770. In practice, this single beam configuration is rarely used due to output drifts as a result of temperature changes, instability of the individual components used, as well as contamination of windows enclosing the gas sample. Therefore, dual wavelength systems have been employed where the first wavelength is absorbed by CO₂, whereas the second wavelength is not absorbed by CO₂ and serves as a reference.

The use of a narrow band gap III-V material for optical absorption gas sensors has been described in US 2013/0271751 A1, WO 2007/091043 A1, and D. Gibson and C. MacGregor, "A Novel Solid State Non-Dispersive Infrared CO2 Gas Sensor Compatible with Wireless and Portable Deployment", Sensors 2013, 13(6):7079-7103 (May 2013) . A gas sensor using a cell structure with increased optical length by multiple reflections is specified in US 4,322,621 and WO 2011/086394 A1.

However, there is a need for improving gas sensing in capnometers.

### SUMMARY OF THE INVENTION

The invention is defined in claim 1 and its dependent claims annexed hereto and provides a capnometer comprising:
an air flow region through which said gas passes to and/or from said patient's lung;
a mid-IR semiconductor emitter (2) configured to provide IR light at a wavelength in the range 3-5 µm;
a mid-IR semiconductor detector (3) to detect said IR light;
a reflector (4) to reflect said IR light emitted by said emitter;
wherein said emitter, said detector and said reflector are arranged such that said IR light emitted by said emitter passes through said air flow region via said reflector to said detector; the air-flow region of the capnometer further comprising:
   a first air-flow path connecting an inhaling/exhaling portion of the capnometer at which a user of the capnometer inhales/exhales air with an intake portion of said capnometer through which air enters said capnometer, and
   a second air-flow path connecting said inhaling/exhaling portion with an exit portion of said capnometer at which air exits said capnometer; and
a diversion device for blocking one of said first and second air-flow paths in the capnometer at a time, wherein said a diverting valve which is controlled by said inhalation/exhalation of said air by said user, whereby during inhalation the diverting valve opens the first air-flow path between the inhalation/exhalation portion and the intake portion while it closes the second air-flow path between the inhalation/exhalation portion and the exit portion and whereby during exhalation the diverting valve opens the second air-flow path between the inhalation/exhalation portion and the exit portion while it closes the first air-flow path between the inhalation/exhalation portion and the intake portion. A capnometer according to the preamble of claim 1 is described in US5261415 A.

A second aspect of the present invention provides a method of calibrating a capnometer, the method comprising:
providing a capnometer according to the first aspect of the present invention;
measuring, using said capnometer, a CO₂ level of air surrounding said capnometer at two points in time before and after an exhaling-inhaling cycle of a user of said capnometer, to provide a first and second CO₂ background level input; and
calibrating said capnometer based on a difference between said first and second CO₂ background level inputs.

The method may include measuring said CO₂ level of air surrounding said capnometer before and after a plurality of said exhaling-inhaling cycles. Air exhaled by said user may exit said capnometer through a first part of the capnometer which is different to a second part of the capnometer through which air inhaled by said user enters said capnometer.

A further aspect of the present invention provides a system comprising:
a capnometer according to the first aspect, the capnometer configured to output gas concentration data from a gas inhaled/exhaled from a patient; and
a data processor configured to receive said gas concentration data, and configured to perform the method according to the second aspect.

The capnometer disclosed herein is for detecting a concentration of a component in a gas, wherein said gas is inhaled and/or exhaled by a patient, said capnometer comprising: an air flow region through which said gas passes to and/or from said patient's lung; a mid-IR semiconductor emitter configured to provide IR light at a wavelength in the range 3-5 µm; a mid-IR semiconductor detector to detect said IR light; a reflector to reflect said IR light emitted by said emitter; wherein said emitter, said detector and said reflector are arranged such that said IR light emitted by said emitter passes through said air flow region via said reflector to said detector.

The capnometer may be used to quantitatively measure the concentration (or associated temporal variation, as will be further described below) of a particular gas component in a gas mixture using the principle of non-dispersive IR absorption.

The reflecting geometry of the capnometer provides for low cost production and low power consumption. The inventors have realised that transmissive optics, rotating elements or beam splitter optics with filters, which are generally employed in a collimated optics geometry, may be made redundant in a capnometer by using the reflecting geometry. The capnometer may therefore be utilised to perform measurements in which an adjacent emitter/detector pair is arranged on one side of a gas sample volume and a reflecting surface is provided on the other side of the gas sample volume.

The reflecting geometry further allows for achieving an appropriate sampling length in order to achieve required signal-to-noise ratio in the detector at the gas concentration appropriate for lung function measurements. In the reflecting geometry, the sampling length may be determined by the distance between the emitter and the reflector as well as the distance between the reflector and the detector.

The capnometer may be operated in a non-diverting or substantially non-diverting mode so that high temporal resolution may be achieved when the concentration of the component in the gas is determined.

The capnometer may be implemented in a way which allows the measurement of a gas concentration close to the oro-nasal orifices.

In a preferred embodiment of the capnometer, the detection of the concentration of the component in a gas comprises detecting a temporal variation of the concentration of the component.

It will be appreciated that a number of configurations may be employed using the reflective geometry.

In a preferred embodiment of the capnometer, the emitter is located at a first location on a first side of said air flow region, the detector is located at a second location on a second side of said air flow region, and wherein said second side is opposite said first side from said air flow region.

Preferably, the reflector is located at a third location on a third side of said air flow region, wherein said third side is adjacent said first and second sides, respectively. The reflector may further be located at a fourth location on a fourth side of said air flow region, wherein said fourth side is opposite said third side from said air flow region. This may allow increasing the signal detected by the detector to improve the signal-to-noise ratio.

Alternatively, a geometry may be used in which the emitter is located at a first location on a first side of said air flow region, wherein said detector is located at a second location on a second side of said air flow region, wherein said first side is adjacent said second side, wherein said reflector is located at a third location on a third side of said air flow region, and wherein said third side is opposite said first side or opposite said second side.

It will be understood that a variety of configurations may be exploited in the reflective geometry of the capnometer. The relative locations of emitter, detector and reflector, and/or the shape of the reflector may be modified in order to ensure that IR light emitted by the emitter passes through the air flow region via the reflector to the detector.

In a preferred embodiment, the capnometer further comprises a breath tube, wherein the breath tube defines a channel between the emitter/detector and the reflector for the air flow region. Preferably, the breath tube is removable from the capnometer. This may be particularly useful since exchanging the breath tube after use may allow for minimising any contamination on the breath tube which could have an undesired impact on the measurements of the gas concentration.

In embodiments of the capnometer which use a breath tube, the emitter, the detector and the reflector may be mounted in the breath tube. Where the breath tube is removable from the capnometer, exchanging the breath tube with emitter, detector and reflector may allow for minimising any contamination on the emitter, detector and reflector.

In another preferred embodiment, the capnometer may further comprise an optical layer between the emitter and the reflector and/or between the detector and the reflector for improving a collection efficiency of the IR light emitted by the emitter onto the detector. This optical layer may comprise mid infrared transmissive material such as, but not limited to silicon. Other materials suitable for the optical layer will be known to those skilled in the art, and include, but are not limited to ZnS, ZnSe, Ge, chalocogenide glasses and certain polymers. It will be appreciated that where emitter and detector are arranged on the same side of the air flow region, the optical layer may be a single layer, or two separate layers between emitter/reflector and detector/reflector, respectively. Layer or layers may have anti-reflection coatings applied to their surfaces, thereby minimising or reducing reflection loss and maximising or increasing transmission through the layer or layers

In a further preferred embodiment of the capnometer, the emitter and the detector are located external to the breath tube, and the breath tube comprises a mid-IR transmissive portion, wherein the mid-IR transmissive portion is aligned with the emitter and the detector to allow mid-IR light to pass therethrough into and out of the breath tube. Preferably, the mid-IR transmissive portion comprises a separate window. It will be understood that if no separate window is exploited, the breath tube may be mid-IR transmissive at the portion which is aligned with the emitter and the detector. Where the mid-IR transmissive portion comprises a separate window, the capnometer preferably further comprises a seal arranged between the breath tube and the separate window. The seal may be suitable for preventing air surrounding the capnometer from penetrating into the air flow region where the gas is sampled.

In a preferred embodiment of the capnometer, the mid-IR transmissive portion comprises an anti-reflection coating and/or an anti-fog coating. This may increase a collection efficiency of IR light emitted by the emitter onto the detector, and may therefore improve the signal-to-noise ratio as the coatings reduce scattering of IR light on the mid-IR transmissive portion. Alternatively, or additionally, the capnometer may further comprise a heater for heating the mid-IR transmissive portion. In this way, water condensation on the mid-IR transmissive portion may be prevented in order to increase the collection efficiency and hence the signal-to-noise ratio.

In addition to the mid-IR transmissive portion, in a preferred embodiment, the capnometer may further comprise an optical layer between the emitter and the reflector and/or between the detector and the reflector for improving a collection efficiency of the IR light emitted by the emitter onto the detector. It will be appreciated that where emitter and detector are arranged on the same side of the air flow region, the optical layer may be a single layer, or two separate layers between emitter/reflector and detector/reflector, respectively. Preferably, the optical layer comprises an anti-reflection coating and/or an anti-fog coating. More preferably, the capnometer may further comprise a second heater for heating the optical layer in order to avoid water condensation on the surface of the optical layer. It will be appreciated that the first heater and second heater may be integral. Furthermore, the mid-IR transmissive portion and the optical layer may, in embodiments, be integral.

As outlined above, the optical layer may comprise silicon, ZnS, ZnSe, Ge, chalocogenide glasses, certain polymers, or other materials known to those skilled in the art.

In embodiments where the emitter and the detector are external to the breath tube, the reflector may be mounted in the breath tube. Preferably, the reflector may comprise a coating on an inner surface of the breath tube, in particular an anti-reflection and/or an anti-fog coating.

It will be understood that a preferable type of coating (anti-reflection coating and anti-fog coating) may be determined dependent on the specific materials used for optical layer, reflector, mid-IR transmissive window and other components which may be in contact with the sampling gas.

In embodiments, the breath tube may comprise one or more alignment features for enabling the arrangement of the reflector with the emitter and the detector. Hence, these alignment features may improve a signal-to-noise ratio and reproducibility of the measurements taken with the capnometer. The one or more alignment features may comprise alignment pins which may provide kinematic location of the capnometer component parts and ease of assembly.

Alternatively, in embodiments of the capnometer, the reflector is located external to the breath tube, and the breath tube comprises a second mid-IR transmissive portion, wherein the second mid-IR transmissive portion is aligned with the reflector to allow mid-IR light to pass therethrough into and out of the breath tube.

In embodiments of the capnometer, the emitter may be configured to provide the IR light at two or more different wavelengths in the range 3-5 µm, and the detector may be configured to detect the two or more different wavelengths for the signal processor. This embodiment may be particularly suitable for detecting different gases with different absorption peaks or generally different absorption spectra.

In embodiments, the reflector may be mounted on an inner surface of the breath tube. The reflector may be integral to the breath tube or integral to a body of the capnometer. Alternatively, the capnometer may further comprise a reflector mounting for supporting the reflector.

In a preferred embodiment, the capnometer further comprises a memory to store the level data output. This may allow for analysing the gas concentration and/or temporal variation of the gas concentration measured with the capnometer at a later stage.

In a preferred embodiment, the gas component to be detected in the gas mixture is CO₂.

In a further preferred embodiment of the capnometer, the emitter comprises a III-V mid-IR semiconductor emitter.

In another preferred embodiment of the capnometer, the detector comprises a III-V mid-IR semiconductor detector.

It will be appreciated that the type of emitter and detector may be determined by the gas and/or gas component to be sampled.

In a preferred embodiment of the capnometer, the reflector comprises a reflective metal film coated with an organic layer. The organic layer may comprise an anti-fog coating, in particular where the reflector is arranged on an inner surface of the breath tube. Alternatively or additionally, a heater, e.g. one of the heaters specified above, may be provided in the capnometer for heating the organic layer to prevent water condensation on a surface of the organic layer.

It will be important to obtain a sufficient temporal resolution of the measurements to allow the signal to convey enough information about a perfusion and a ventilation of lungs of a patient. The sample width may be reduced using cut down optics and/or a breath tube design to accelerate gas through the sampling area. Therefore, in a preferred embodiment of the capnometer, the air flow region may have a cross-sectional area which is reduced where the IR light passes through the air flow region.

The capnometer may be used in combination with a medical device, wherein the capnometer further comprises blocking means to inhibit air surrounding the capnometer from flowing into the medical device.

In a preferred embodiment of the capnometer, the emitter comprises a plurality of emitters, wherein each of the plurality of emitters emits light centred at a different, respective wavelength. This may allow different gases with different absorption spectra to be sampled with the capnometer. Furthermore, this embodiment allows for calibrating the device and/or determining when a measurement of a gas concentration is void, as will be further described below.

In a further preferred embodiment, the detector comprises a plurality of detectors, wherein each of the plurality of detectors detects light at a different, respective range of wavelengths.

The design of the optical path is to ensure a good collection efficiency of the optical system, comprising emitter and detector, employed in the capnometer. The use of materials with low absorption at wavelengths where the component absorbs light (e.g. 4.26 µm for CO₂) may be preferable. Moreover, a high reflective surface of the reflector may be achieved by using, e.g. Au deposited by physical vapour deposition or electrochemical deposition.

The capnometer may be required to be calibrated in order for the output of the device, i.e. a CO₂ concentration, to meet accuracy requirements of a gas monitoring device, for example for medical application. Calibration may be performed in different ways, for example by providing a known gas concentration in the sampling area.

Although the signal which may be received by a detector in the capnometer may be affected by the CO₂ concentration between it and the emitter, the signal may also be affected by other absorbing and scattering materials in the path between emitter and detector, and/or drift in the emitter output and/or drift in the detector electronics. A reference channel may be used to compensate for these factors, which may take the form of an additional detector measuring at a wavelength to one side of the CO₂ absorption band that may be similarly affected by the same factors affecting the CO₂-centred detector. The inventors have realised that it is advantageous to avoid having to use a reference channel as regular zeroing may still be required as there may still be differences between the two channels.

Therefore, in a related aspect of the present invention, there is provided a method of calibrating a capnometer, the method comprising: providing a capnometer according to the present invention, measuring, using said capnometer, a CO₂ level of air surrounding said capnometer at two points in time before and after an exhaling-inhaling cycle of a user of said capnometer, to provide a first and second CO₂ background level input; and calibrating said capnometer based on a difference between said first and second CO₂ background level inputs.

We note that "user" and "patient" are used synonymously throughout the specification.

A capnometer using reflective optics may provide for a longer path length between emitter and detector and inherent stability of the emitter. These factors may allow realisation of a capnometer which may accurately measure CO₂ concentration at atmospheric background levels of CO₂.

For a capnometer which may be used as a personal medical or diagnostic device where the period of operation may be a few minutes long, it may be possible to measure the background CO₂ level at the beginning and end of the sample period and a difference in value may be due to some factor other than the CO₂ level.

By measuring the change in background over time, a correction may be applied to the capnometer CO₂ output.

In preferred embodiments, the background level is measured during the breath cycle when fresh air is drawn into the device. In this way, the device may be calibrated whilst being used.

In a preferred embodiment of the method, the CO₂ level of air surrounding the capnometer is measured before and after a plurality of the exhaling-inhaling cycles. This may advantageously allow for determining a CO₂ background level drift which may occur only slowly in time. The slow drift may be detected and the capnometer may be calibrated accordingly.

It will be appreciated that the CO₂ background level may be determined in between each exhaling-inhaling breath cycle by the user over a longer period of time, for example from the beginning of the use of the capnometer by the user to the end, i.e. the end of the last breath cycle. This may allow for calibrating the capnometer based on both sudden changes in the CO₂ background level and changes in the CO₂ background level which may occur over a (relatively) longer period of time.

In embodiments, the CO₂ background level is measured at different points in time after a predetermined number of exhaling-inhaling cycles has lapsed. Alternatively, the CO₂ background level may be measured with a predetermined time period in between the measurements, i.e. independent from the number of breath cycles.

In a further preferred embodiment of the method, air exhaled by the user exits the capnometer through a first part of the capnometer which is different to a second part of the capnometer through which air inhaled by the user enters the capnometer. This may be particularly useful when the CO₂ background level is measured before and after a single (or a few) exhaling-inhaling cycles. Embodiments therefore allow for minimising or reducing the deadspace (i.e. space in which exhaled air by the user is not entirely replaced with fresh inhaled air) in the capnometer so that the capnometer is fully (or almost fully) flushed with fresh inhaled air.

In order to achieve the above, the capnometer comprises a diversion device.

The diversion device comprises a diverting valve which is controlled by the inhalation/exhalation of air by the user. During inhalation, the diverting valve opens the first air-flow path between the inhaling/exhaling portion and the intake portion of the capnometer, while it closes the second air-flow path between the inhaling/exhaling portion and the exit portion of the capnometer at the same time. The situation is reversed during exhaling by the user, such that the connection between the inhaling/exhaling portion and the intake portion is closed, while the connection between the inhaling/exhaling portion and the exit portion is open. It will be appreciated that the opposite operation of the diverting valve may be employed, in which the first air-flow path is blocked during inhalation and the second air flow-path is open (and opposite during exhaling). The diverting valve allows for a simple, reliable and cheap construction of the diversion device.

It will be understood that the diversion device may take other forms, which may be controlled by other means, such as electronic means.

It will be understood that two or more of the calibration methods described herein may be combined, in particular to provide for a more precise calibration of the capnometer.

Embodiments of the capnometer described herein may be combined with and/or incorporated into an inhaler. Therefore, there is provided an inhaler comprising a capnometer as described in any of the embodiments herein, wherein said capnometer is configured to monitor a CO₂ level in air inhaled through or exhaled through said inhaler.

In a further related aspect of the present invention, there is provided a system comprising a capnometer as described in embodiments herein, the capnometer configured to output gas concentration data from a gas inhaled/exhaled from a patient; and a data processor configured to receive the gas concentration data, and configured to perform any one of the methods according to embodiments described herein.

The gas concentration data may, in embodiments, be CO₂ gas concentration data.

It will be understood that the processor for receiving the gas concentration data and for performing any one (or more) of the methods described herein may be integral to the capnometer.

### BRIEF DESCRIPTION OF THE DRAWINGS

These and other aspects of the invention will now be further described by way of example only, with reference to the accompanying figures, wherein like numerals refer to like parts throughout, and in which:
Figure 1 shows a schematic illustration of components of a capnometer falling outside the scope of the present invention;
Figure 2 shows a schematic cross-sectional view of components of a capnometer falling outside the scope of the present invention;
Figure 3 shows a schematic of a capnometer comprising a silicon optic window falling outside the scope of the present invention;
Figure 4 shows a schematic illustration of a capnometer comprising an IR-transmissive window falling outside the scope of the present invention;
Figure 5 shows a schematic illustration of a capnometer incorporating a disposable breath tube falling outside the scope of the present invention;
Figure 6 shows a schematic illustration of a capnometer falling outside the scope of the present invention;
Figure 7 shows CO₂ concentration as a function of time;
Figure 8 shows CO₂ concentration as a function of time and indications when CO₂ background level measurements are taken in this example; and
Figure 9 shows a schematic of parts of a capnometer according to the present invention, the capnometer comprising a diverting valve.

### DETAILED DESCRIPTION OF PREFERRED EMBODIMENTS

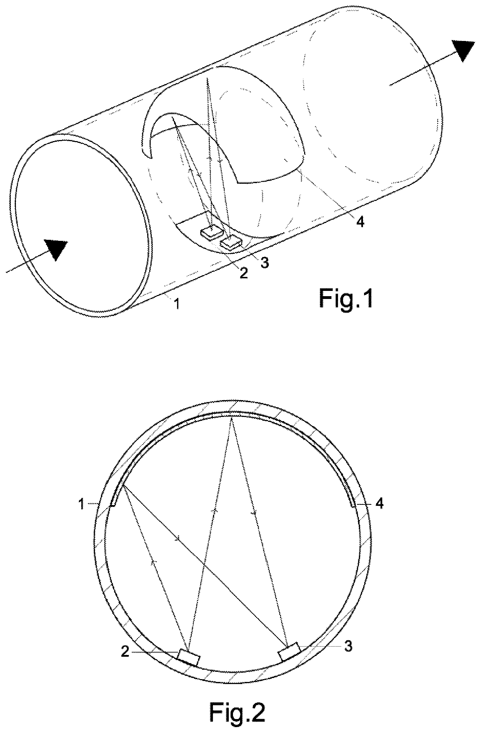

Figure 1 shows a schematic illustration of components of a capnometer according to an example not forming part of the present invention. The device may be exploited to measure the concentration of a gas component in a sample gas flowing across an air flow region interposed between an emitter (2)/detector (3) pair and a reflector (4). In this example, the emitter (2)/detector (3) pair and the reflector (4) are incorporated into a breath tube (1) and may be connected to a suitable electronic drive and detecting circuitry. The value of the detector current may be proportional to the emitter current and the amount of gas to be sampled in the sampling area.

The emitter (2) and/or the detector (3) of the capnometer may be composed of III-V semiconductors. It will be appreciated that materials suitable for emitter (2) and detector (3) will be known to those skilled in the art. In this example, an emitter diode is designed to emit IR radiation centred around 4.26 µm and the detector (3) is a photo-diode which has a peak sensitivity centred around 4.26 µm.

The path length traversed by the IR light emitted by the emitter (2) and detected by the detector (3) via the reflector (4) may be chosen such that the attenuation of the signal by absorption by CO₂ molecules is such that a change in intensity can be detected by the detector (3) for the concentration range that is appropriate for the gas stream. For human breath, the range of CO₂ concentration varies between the background CO₂ level which is inhaled by the human and up to 5% of gas which is exhaled by the human. In this case, the distance traversed by the IR light may be approximately 20 mm.

As illustrated in the cross-sectional view of Figure 2, the width of the sampled area may be determined by the distance between emitter (2)/reflector (4) and reflector (4)/detector (3). The width of the sampled area may be chosen such that the temporal resolution of the system is high enough for the intended application of the device. As outlined above, the air flow region may have a cross-sectional area which is reduced where the IR light passes through the air flow region to increase a time-resolution of the determination of the concentration of the gas component to be determined.

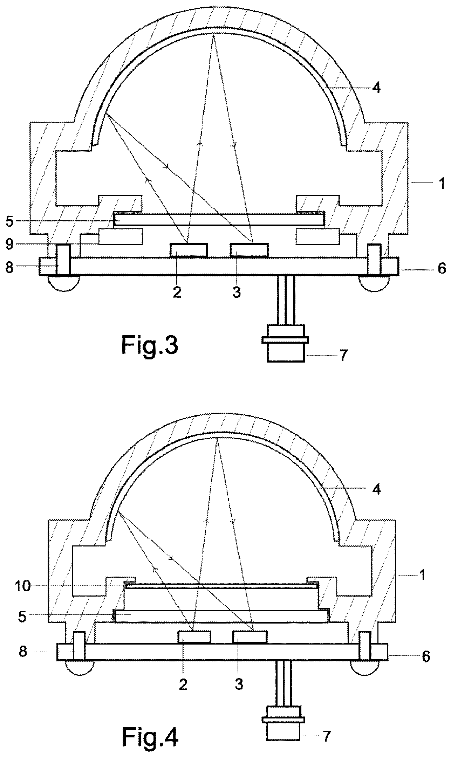

Figure 3 shows a capnometer comprising a silicon optic window (5) according to examples not forming part of the present invention. The capnometer may be suitable for a single use. Alternatively, the capnometer may incorporate a disposable breath tube (1) with a reflector (4) and an optical layer (5) which may comprise silicon.

The breath tube (1) may be made from a high-density polymer which may have been injection moulded to form a reflector shape. The breath tube (1) may be coated, in this example by evaporation or via an electrochemical method, with a thin metal film which forms a reflector (4) with high reflection efficiency. It will be appreciated that a variety of deposition techniques known to the skilled person may be exploited. In this example, the high-density polymer breath tube (1) is coated with a gold film. It will be understood that a variety of materials known to those skilled in the art may be exploited for the breath tube (1) and the reflector (4), such as, but not limited to aluminium and silver.

The breath tube (1) may contain channels which allow a sample gas to be diverted from the air flow region where the sample gas is examined, so that a conductance may be achieved which is high enough for the flow to be sampled.

In the example shown in Figure 3, an optical layer (5) which may comprise silicon is provided opposite the reflector (4). The optical layer (5) may form a seal with the breath tube (1). The purpose of the optical layer (5) comprising silicon is both to protect the emitter (2)/detector (3) pair from breath, e.g. to avoid a contamination of the emitter (2)/detector (3) pair, and to increase the collection efficiency of the system by directing more light emitted from the emitter (2) to impinge on the detector (3). Other materials suitable for the optical layer (5) will be known to those skilled in the art, and include, but are not limited to ZnS, ZnSe, Ge, chalocogenide glasses and certain polymers. In this example, a gap is provided between the optical layer (5) and the emitter (2)/detector (3) pair. It will be appreciated that the optical layer (5) may alternatively be in contact with the emitter (2)/detector (3) pair. Furthermore, the optical layer (5) may comprise a plurality of layers between emitter (2) and reflector (4) and/or between detector (3) and reflector (4), respectively.

The capnometer may further comprise a seal (9) to prevent gas from the surrounding area from entering the sampling area. In this example, the seal (9) is arranged at two locations where the breath tube (1) and the optical layer (5) connect with each other. It will be appreciated that the seal (9) may be arranged at one or more locations. The seal (9) may be arranged on a side facing the air flow region where the breath tube (1) and the optical layer (5) connect, and/or on a side facing away from the air flow region (as shown in Figure 3).

As outlined above, the optical layer (5) may comprise an anti-reflection coating to minimise losses at interfaces with air.

Surfaces of the reflector (4) and the optical layer (5) which are in contact with the sample gas may be coated with materials which modify the surface energy. Hence, when water condensate from breath condenses on these surfaces, in the case of high surface energy modifiers, the water condensate forms a thin film rather than a droplet or droplets which potentially scatter and therefore reduce the IR radiation impinging on the detector (3). High surface energy materials for coating the surfaces may be, but are not limited to, hydroxyl and carboxyl containing hydrocarbon thiols. Hydroxyl and carboxyl containing hydrocarbon thiols may be particularly suitable for coating a gold surface to form a self-assembled mono-layer. Poly-ethylene oxide or an amine of cyano containing polymers may be deposited on the surfaces of both the reflector (4) and the optical layer (5). In the case of low surface energy modifiers, the water condensate forms droplets that bead up and fall from the window, or in the case of the reflector (4) coat less of the reflector (4) and so have less effect on the signal. Low surface energy materials may be, but are not limited to fluoro-carbon containing molecules. Fluoro-carbon thiols may be particularly suitable for coating a gold surface using a self-assembled mono-layer.

In addition or in place of surface energy modifying materials, the reflector (4) and/or optical layer (5) which are in contact with the sample gas may be heated so that the surface temperature is high enough to prevent condensation on the reflector (4) and/or the optical layer (5).

The distance between the optical layer (5) and the emitter (2)/detector (3) pair may be kept as small as practical in orderto minimise any sampling of gas surrounding the breath tube (1). Preferably, this distance may be less than 1 cm, more preferably less than 1 mm, or even more preferably less than 0.1 mm. In this example, this distance is typically less than 0.1 mm. In this example, a gap is provided between the optical layer (5) and the emitter (2)/detector (3) pair. It will be appreciated that the optical layer (5) may alternatively be in contact with the emitter (2)/detector (3) pair. Furthermore, the optical layer (5) may comprise a plurality of layers between emitter (2) and reflector (4) and/or between detector (3) and reflector (4), respectively.

The emitter (2)/detector (3) pair may be mounted on a printed circuit board (6) which may incorporate high precision locating lugs for locating pins (8) that are incorporated into the injection moulded breath tube (1). This may allow for a high precision alignment of the emitter (2)/detector (3) pair to the reflector (4) so that high collection efficiency may be achieved.

The printed circuit board (6) may allow for a connection between the emitter (2)/detector (3) pair and the driving circuit.

An electrical connector (7) may be connected to the printed circuit board (6) in order to drive the emitter (2)/detector (3) pair.

The system may be designed such that the whole assembly may be removed from the driving electronics and body of the capnometer, and may be replaced. Therefore, the breath tube assembly comprising reflector (4), optical layer (5) and emitter (2)/detector (3) printed on the circuit board (6) may be disposed after use of the capnometer.

Figure 4 shows a schematic of a capnometer comprising an IR-transmissive window (10) according to examples not forming part of the present invention. We note that any references to IR-transmissive of any of the components described herein comprises IR-transparent. The IR-transmissivity of any of the component may be above a pre-defined threshold.

In this example, the device comprises an injection moulded breath tube (1) with a coating to form a reflector (4).

Furthermore, in this example, opposite the reflector (4) there is an opening which is covered by a window (10) which has a low attenuation to IR radiation. Materials for such an IR-transmissive window (10) include, but are not limited to, polyolefins such as polyethylene. The thickness of the window (10) is preferably less than 1 cm, more preferably less than 1 mm, or even more preferably less than 100 µm. In this example, the thickness of the IR-transmissive window (10) is less than 100 µm.

The breath tube (1) with reflector (4) and thin IR-transmissive window (10) may incorporate pins (8) which may locate with the printed circuit board (6) containing the emitter (2)/detector (3) pair covered with an optical layer (5) comprising silicon. This may allow for the breath tube (1) to be removable from the capnometer. In this manner, a lower-cost disposable breath tube (1) may be manufactured. It will be appreciated that additionally or alternatively, locator pins (8) may be incorporated into the printed circuit board (6).

An electrical connector (7) may be connected to the printed circuit board (6) in order to drive the emitter (2)/detector (3) pair.

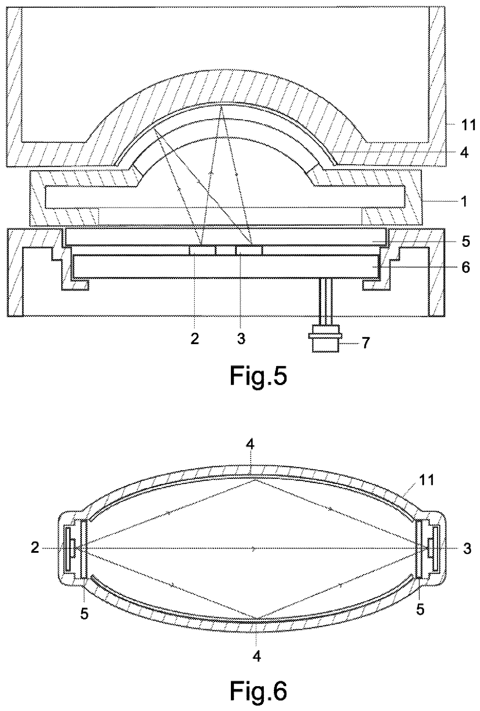

Figure 5 shows a schematic of a capnometer incorporating a disposable breath tube (1). In this example not forming part of the invention, the capnometer comprises a body (11) of injection moulded high density polymer. The body (11) may contain locating pins (not shown) which align to lugs in the printed circuit board (6). The reflector (4) may optionally be integral to the body (11).

The emitter (2)/detector (3) pair may be arranged on top of the printed circuit board (6). An electrical connector (7) may be connected to the printed circuit board (6) in order to drive the emitter (2)/detector (3) pair.

In this example, an optical layer (5) comprising silicon is disposed on top of the emitter (2)/detector (3) pair.

A breath tube (1) may be interspersed between the moulded body (11) and the printed circuit board (6). The breath tube (1) may comprise windows or IR-transmissive or IR-transparent material. Therefore, the breath tube (1) in this example may be disposable, allowing for a low-cost production of the breath tube (1).

Figure 6 shows a schematic of a capnometer wherein the emitter (2) and detector (3) are arranged on opposite sides of the air flow region. This example which does not form part of the invention exploits a reflecting geometry, wherein the emitter (2), the detector (3) and the reflector (4) are arranged such that IR light emitted by the emitter (2) passes through the air flow region via the reflector (4) to the detector (3).

In this configuration, the reflector (4) may be arranged on one side of the air flow region only, or, alternatively, on both sides of the air flow region as shown in Figure 6.

Furthermore, the example of Figure 6 may allow using either a reflecting geometry, or a non-reflecting geometry in which IR light passes through the air flow region without being reflected by the reflector (4), or a combination of reflecting and non-reflecting geometries.

In this example, emitter (2) and detector (3) may be mounted in a breath tube (not shown in Figure 6), which may be removable from the capnometer. Alternatively, emitter (2) and detector (3) may be mounted on two inner surfaces of the body (11) of the capnometer on opposing sides facing the air flow region. Emitter (2) and detector (3) may be integral to the body (11) of the capnometer.

As shown in Figure 6, optical layers (5) which may comprise silicon, may be arranged between the air flow region and the emitter (2) and the detector (3), respectively. It will be appreciated that only a single optical layer (5) may be arranged, either between the air flow region and the emitter (2), or between the air flow region and the detector (3). In this example, optical layers (5) are arranged between both the air flow region and the emitter (2) as well as the air flow region and the detector (3). As outlined above, (an) optical layer(s) (5) may improve a collection efficiency of IR light emitter by the emitter (2) onto the detector (3).

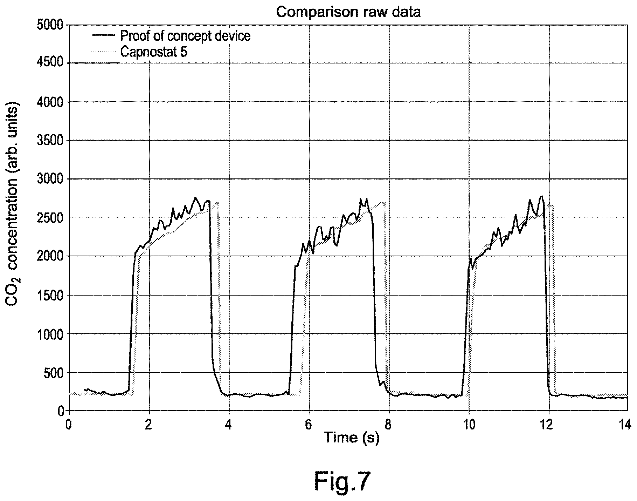

Figure 7 shows measurements of CO₂ concentration as a function of time performed with a capnometer as described in examples herein, as well as with a standard Capnostat ^{®} 5 capnometer, respectively. In this example, three inspiration/expiration cycles of a patient have been recorded with each of the above-specified devices over a time period of 14 seconds.

It can be seen that measurements taken with the capnometer described herein show some minor fluctuations in the detected CO₂ concentration compared to the slightly more stable Capnostat ^{®} 5 capnometer. However, generally, the signals detected with each of the capnometers substantially coincide with regard to their frequency, amplitude, rise and fall time of a signal, and other characteristics. This example shows a proof of concept of the capnometer described in this specification.

In examples described herein, there is a problem of contamination of the reflective surface and/or any IR-transmissive windows/components. Contamination may reduce the signal received by the detector (3) and it may appear as an increase in CO₂ level even if no change has actually occurred. One method to mitigate this is to monitor the inspiration phase of the breath cycle where fresh air with low CO₂ levels is passed through the sampling area. Typically, the CO₂ concentration is approximately 450 ppm. It will be appreciated that the threshold may vary and may preferably be adjusted depending on conditions of the area surrounding the device. If the measured CO₂ level is higher than the threshold, the system may indicate that the measurement is void. A correction to the measured CO₂ level may be made.

In an alternative example not forming part of the invention, the capnometer comprises an additional emitter (2) which emits radiation centred around less or more than 4.26 µm, but still within the detecting range of the detector (3). The two emitters (2) may be pulsed in opposite phases so that the detector (3) may detect the two emitters (2) at separate points in time. In this way, any scattering of radiation due to contamination may be similar between the two emitter radiations. A correction may be applied to the signal from the emitter (2) emitting IR-light centred at or around 4.26 µm. It will be appreciated that alternatively or additionally, the signal detected by the detector (3) may be corrected based on the determined contamination.

In examples described herein, there is a problem of achieving accurate calibration of the system. This is particularly the case where a replaceable breath tube (1) is employed since there is a possibility of small variations in alignment. The device may be calibrated assuming the background CO₂ level is known. This might be the case if the air is ambient air. Alternatively, the CO₂ level may be obtained from a different information source.

Alternatively, a breath tube (1) incorporating a gas of known CO₂ level may be used for calibrating the device. The breath tube (1) may comprise end caps which may have removable elements such that after calibration the removable elements may be peeled off and the device is ready to use.

The effect of an incorrect zero value may have a large impact on the calculated CO₂ concentration at end tidal values found in human breath - typically around 5%. For example, an error of approximately 0.3% in the signal level at the zeroing point will cause a ~10% variation in the calculated output at 5% CO₂ concentration. Therefore, if a change to the signal of the capnometer occurred due to any other factor apart from the CO₂ concentration, that causes the output as measured at the original zeroing point to change by more than 0.3%, then larger than 10% errors may occur in the value of end tidal CO₂ concentration. Hence, in order to achieve an accurate capnogram without a reference channel, it is important to monitor the stability of the CO₂ background level.

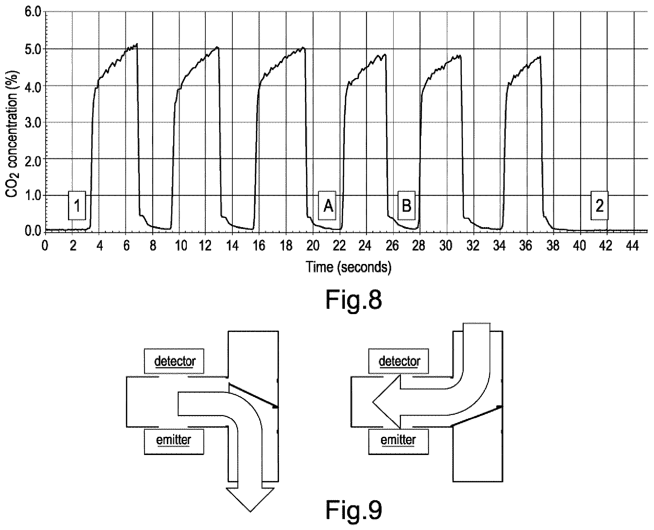

Figure 8 shows CO₂ concentration as a function of time. A typical capnogram of human breathing is indicated.

Before breathing starts, there is a flat baseline level and zeroing can be made at this point (point 1 in Figure 8). The breathing then starts with the typical breath profile showing a rapid rise in CO₂ concentration to the end tidal plateau, the highest point being the end tidal peak at around 5% before inhalation and a rapid decrease back to a background level occurs.

After breathing has stopped, the device should go back to the same background level as at the start (if no change in CO₂ background level has occurred), which is recorded at this point (point 2 in Figure 8). However, if the background level is different at points 1 and 2, this indicates a drift in the CO₂ background level of air surrounding the capnometer. By making the assumption that, for example the drift occurred linearly over the breath sample, a correction can be made to the capnogram by carrying out a linear correction.

Figure 9 shows a schematic of parts of a capnometer according to the present invention which includes a diverting valve.

The value of the CO₂ background level can be made, for example, between breaths at points A and B (see Figure 8). A correction can be applied based on the CO₂ background level change that occurs between these points. For this to occur, the deadspace (space in the capnometer at which air exhaled by patient is not entirely or nearly entirely replaced subsequently by air inhaled by the user) in the capnometer needs to be minimised so that the capnometer is fully (or at least almost fully) flushed with inhaled air. In this embodiment, a diverting valve is placed in the output of the capnometer so that air can be inhaled from the opposite side to the exhalation, as shown in Figure 9, to reduce the impact of inhaling exhaled air.

No doubt many other effective alternatives will occur to the skilled person. It will be understood that the invention is not limited to the described embodiments and encompasses modifications apparent to those skilled in the art and lying within the scope of the claims appended hereto.

## Claims

1. A capnometer comprising:
an air flow region through which said gas passes to and/or from said patient's lung;
a mid-IR semiconductor emitter (2) configured to provide IR light at a wavelength in the range 3-5 µm;
a mid-IR semiconductor detector (3) to detect said IR light;
a reflector (4) to reflect said IR light emitted by said emitter;
wherein said emitter, said detector and said reflector are arranged such that said IR light emitted by said emitter passes through said air flow region via said reflector to said detector; the airflow region of the capnometer further comprising:
a first air-flow path connecting an inhaling/exhaling portion of the capnometer at which a user of the capnometer inhales/exhales air with an intake portion of said capnometer through which air enters said capnometer, and **characterised by**
a second air-flow path connecting said inhaling/exhaling portion with an exit portion of said capnometer at which air exits said capnometer; and
a diversion device for blocking one of said first and second air-flow paths in the capnometer at a time, wherein said diversion device comprises a diverting valve which is controlled by said inhalation/exhalation of said air by said user, whereby during inhalation the diverting valve opens the first air-flow path between the inhalation/exhalation portion and the intake portion while it closes the second air-flow path between the inhalation/exhalation portion and the exit portion and whereby during exhalation the diverting valve opens the second air-flow path between the inhalation/exhalation portion and the exit portion while it closes the first air-flow path between the inhalation/exhalation portion and the intake portion.

2. A capnometer as claimed in claim 1, wherein said emitter (2) is located at a first location on a first side of said air flow region, wherein said detector (3) is located at a second location on a second side of said air flow region, wherein said first side is adjacent said second side, wherein said reflector (4) is located at a third location on a third side of said air flow region, and wherein said third side is opposite said first side or opposite said second side.

3. A capnometer as claimed in any preceding claim, further comprising a breath tube (1), wherein said breath tube defines a channel between said emitter/detector (2,3) and said reflector (4) for said air flow region, preferably wherein said breath tube is removable from said capnometer.

4. A capnometer as claimed in claim 3, wherein said emitter (2), said detector (3) and said reflector (4) are mounted in said breath tube (1).

5. A capnometer as claimed in claim 4, further comprising an optical layer (5) between said emitter (2) and said reflector (4) and/or between said detector (3) and said reflector (4) for improving a collection efficiency of said IR light emitted by said emitter onto said detector, preferably wherein said optical layer comprises silicon and/or wherein said optical layer includes a heater.

6. A capnometer as claimed in claim 3, wherein said emitter (2) and said detector (3) are located external to said breath tube (1), and wherein said breath tube comprises a mid-IR transmissive portion (10), said mid-IR transmissive portion being aligned with said emitter and said detector to allow mid-IR light to pass therethrough into and out of said breath tube (1), preferably wherein a heater is provided for heating said mid-IR transmissive portion.

7. A capnometer as claimed in 6, wherein said reflector (4) is located external to said breath tube (1), and wherein said breath tube comprises a second mid-IR transmissive portion (10), said second mid-IR transmissive portion being aligned with said reflector to allow mid-IR light to pass therethrough into and out of said breath tube.

8. A capnometer as claimed in any preceding claim, wherein said emitter (2) comprises a III-V mid-IR semiconductor emitter and/or wherein said detector (3) comprises a III-V mid-IR semiconductor detector.

9. A capnometer as claimed in any preceding claim, wherein said air flow region has a cross-sectional area which is reduced where said IR light passes through said air flow region.

10. A capnometer as claimed in any preceding claim in combination with a medical device, said capnometer further comprising blocking means to inhibit air surrounding said capnometer from flowing into said medical device.

11. An inhaler comprising a capnometer according to any one of the preceding claims, wherein said capnometer is configured to monitor a CO₂ level in air inhaled through or exhaled through said inhaler.

12. A method of calibrating a capnometer, the method comprising:
providing a capnometer as claimed in any one of claims 1 to 10;
measuring, using said capnometer, a CO₂ level of air surrounding said capnometer at two points in time before and after an exhaling-inhaling cycle of a user of said capnometer, to provide a first and second CO₂ background level input; and
calibrating said capnometer based on a difference between said first and second CO₂ background level inputs.

13. A method as claimed in claim 12, wherein said CO₂ level of air surrounding said capnometer is measured before and after a plurality of said exhaling-inhaling cycles.

14. A method as claimed in claim 12 or 13, wherein air exhaled by said user exits said capnometer through a first part of the capnometer which is different to a second part of the capnometer through which air inhaled by said user enters said capnometer.

15. A system comprising:
a capnometer according to any one of claims 1 to 10, the capnometer configured to output gas concentration data from a gas inhaled/exhaled from a patient; and
a data processor configured to receive said gas concentration data, and configured to perform the method according to claims 12 to 14.

## Patentansprüche

1. Kapnometer, das Folgendes umfasst:
einen Luftstrombereich, durch den das Gas zu und/oder von der Lunge des Patienten gelangt;
einen Halbleiter-Emitter für mittleres IR (2), der zur Bereitstellung von IR-Licht von einer Wellenlänge in dem Bereich 3-5 µm konfiguriert ist;
einen Halbleiter-Detektor für mittleres IR (3) zur Detektion des IR-Lichts;
einen Reflektor (4) zur Reflexion des IR-Lichts, das von dem Emitter emittiert wird;
wobei der Emitter, der Detektor und der Reflektor derart angeordnet sind, dass das IR-Licht, das von dem Emitter emittiert wird, durch den Luftstrombereich über den Reflektor zu dem Detektor gelangt; wobei der Luftstrombereich des Kapnometers weiter Folgendes umfasst:
einen ersten Luftstromweg, der ein Einatmungs-/Ausatmungsteil des Kapnometers, an dem ein Anwender des Kapnometers Luft einatmet/ausatmet, mit einem Einlassteil des Kapnometers, durch das Luft in das Kapnometer eintritt, verbindet, und **gekennzeichnet ist durch**
einen zweiten Luftstromweg, der das Einatmungs-/Ausatmungsteil mit einem Auslassteil des Kapnometers, an dem Luft aus dem Kapnometer austritt, verbindet; und
eine Ablenkungsvorrichtung für die Blockierung eines des ersten und zweiten Luftstromwegs in dem Kapnometer zu einem Zeitpunkt, wobei die Ablenkungsvorrichtung ein Umschaltventil umfasst, das durch das Einatmen/Ausatmen der Luft durch den Anwender gesteuert wird, wodurch während der Einatmung das Umschaltventil den ersten Luftstromweg zwischen dem Einatmungs-/Ausatmungsteil und dem Einlassteil öffnet, während es den zweiten Luftstromweg zwischen dem Einatmungs-/Ausatmungsteil und dem Auslassteil schließt, und wodurch während der Ausatmung das Umschaltventil den zweiten Luftstromweg zwischen dem Einatmungs-/Ausatmungsteil und dem Auslassteil öffnet, während es den ersten Luftstromweg zwischen dem Einatmungs-/Ausatmungsteil und dem Einlassteil schließt.

2. Kapnometer nach Anspruch 1, wobei sich der Emitter (2) an einer ersten Stelle auf einer ersten Seite des Luftstrombereichs befindet, wobei sich der Detektor (3) an einer zweiten Stelle auf einer zweiten Seite des Luftstrombereichs befindet, wobei die erste Seite der zweiten Seite benachbart ist, wobei sich der Reflektor (4) an einer dritten Stelle auf einer dritten Seite des Luftstrombereichs befindet und wobei die dritte Seite der ersten Seite gegenüberliegt oder der zweiten Seite gegenüberliegt.

3. Kapnometer nach einem vorstehenden Anspruch, das weiter ein Atemröhrchen (1) umfasst, wobei das Atemröhrchen einen Kanal zwischen dem Emitter/Detektor (2, 3) und dem Reflektor (4) für den Luftstrombereich definiert, bevorzugt wobei das Atemröhrchen von dem Kapnometer entfernbar ist.

4. Kapnometer nach Anspruch 3, wobei der Emitter (2), der Detektor (3) und der Reflektor (4) in dem Atemröhrchen (1) angebracht sind.

5. Kapnometer nach Anspruch 4, das weiter eine optische Schicht (5) zwischen dem Emitter (2) und dem Reflektor (4) und/oder zwischen dem Detektor (3) und dem Reflektor (4) zur Verbesserung einer Sammeleffizienz des IR-Lichts, das von dem Emitter emittiert wird, auf den Detektor umfasst, bevorzugt wobei die optische Schicht Silicium umfasst und/oder wobei die optische Schicht ein Heizelement einschließt.

6. Kapnometer nach Anspruch 3, wobei sich der Emitter (2) und der Detektor (3) außerhalb des Atemröhrchens (1) befinden und wobei das Atemröhrchen ein für mittleres IR durchlässiges Teil (10) umfasst, wobei das für mittleres IR durchlässige Teil mit dem Emitter und dem Detektor ausgerichtet ist, um zu ermöglichen, dass mittleres IR-Licht dahindurch in und aus dem Atemröhrchen (1) gelangt, bevorzugt wobei ein Heizelement für das Erhitzen des für mittleres IR durchlässigen Teils bereitgestellt ist.

7. Kapnometer nach Anspruch 6, wobei sich der Reflektor (4) außerhalb des Atemröhrchens (1) befindet und wobei das Atemröhrchen ein zweites, für mittleres IR durchlässiges Teil (10) umfasst, wobei das zweite, für mittleres IR durchlässige Teil mit dem Reflektor ausgerichtet ist, um zu ermöglichen, dass mittleres IR-Licht dahindurch in und aus dem Atemröhrchen gelangt.

8. Kapnometer nach einem vorstehenden Anspruch, wobei der Emitter (2) einen III-V-Halbleiter-Emitter für mittleres IR umfasst und/oder wobei der Detektor (3) einen III-V-Halbleiter-Detektor für mittleres IR umfasst.

9. Kapnometer nach einem vorstehenden Anspruch, wobei der Luftstrombereich eine Querschnittsfläche aufweist, die verringert ist, wo das IR-Licht durch den Luftstrombereich gelangt.

10. Kapnometer nach einem vorstehenden Anspruch in Kombination mit einer medizinischen Vorrichtung, wobei das Kapnometer weiter Blockierungsmittel umfasst, um zu verhindern, dass Luft, die das Kapnometer umgibt, in die medizinische Vorrichtung strömt.

11. Inhalator, der ein Kapnometer nach einem der vorstehenden Ansprüche umfasst, wobei das Kapnometer konfiguriert ist, um einen CO₂-Gehalt in der Luft, die durch den Inhalator eingeatmet oder ausgeatmet wird, zu überwachen.

12. Verfahren zur Kalibrierung eines Kapnometers, wobei das Verfahren Folgendes umfasst:
Bereitstellen eines Kapnometers nach einem der Ansprüche 1 bis 10;
Messen unter Verwendung des Kapnometers eines CO₂-Gehalts von Luft, die das Kapnometer umgibt, zu zwei Zeitpunkten vor und nach einem Ausatmungs-Einatmungs-Zyklus eines Anwenders des Kapnometers, um einen ersten und zweiten CO₂-Hintergrundgehalteingang bereitzustellen; und
Kalibrieren des Kapnometers basierend auf einem Unterschied zwischen dem ersten und zweiten CO₂-Hintergrundgehalteingang.

13. Verfahren nach Anspruch 12, wobei der CO₂-Gehalt von Luft, die das Kapnometer umgibt, vor und nach einer Vielzahl der Ausatmungs-Einatmungs-Zyklen gemessen wird.

14. Verfahren nach Anspruch 12 oder 13, wobei Luft, die von dem Anwender ausgeatmet wird, aus dem Kapnometer durch einen ersten Teil des Kapnometers austritt, der sich von einem zweiten Teil des Kapnometers unterscheidet, durch den Luft, die von dem Anwender eingeatmet wird, in das Kapnometer eintritt.

15. System, das Folgendes umfasst:
ein Kapnometer nach einem der Ansprüche 1 bis 10, wobei das Kapnometer zur Ausgabe von Gaskonzentrationsdaten von einem Gas, das von einem Patienten eingeatmet/ausgeatmet wird, konfiguriert ist; und
einen Datenprozessor, der zum Empfang der Gaskonzentrationsdaten konfiguriert ist und zur Durchführung des Verfahrens nach den Ansprüchen 12 bis 14 konfiguriert ist.

## Revendications

1. Capnomètre comprenant :
une région d'écoulement d'air à travers laquelle ledit gaz passe vers et/ou depuis le poumon dudit patient ;
un émetteur à semi-conducteur dans l'infrarouge moyen (2) configuré pour fournir une lumière IR à une longueur d'onde comprise entre 3 et 5 µm ;
un détecteur à semi-conducteur dans l'infrarouge moyen (3) pour détecter ladite lumière IR;
un réflecteur (4) pour réfléchir ladite lumière IR émise par ledit émetteur ;
dans lequel ledit émetteur, ledit détecteur et ledit réflecteur sont disposés de telle sorte que ladite lumière IR émise par ledit émetteur passe à travers ladite région d'écoulement d'air par l'intermédiaire dudit réflecteur vers ledit détecteur ; la région d'écoulement d'air du capnomètre comprenant en outre :
un premier chemin d'écoulement d'air qui connecte une partie d'inspiration/expiration du capnomètre au niveau de laquelle un utilisateur du capnomètre inspire/expire de l'air avec une partie d'admission dudit capnomètre par laquelle l'air pénètre dans ledit capnomètre, et **caractérisé par**
un second chemin d'écoulement d'air qui connecte ladite partie d'inspiration/expiration à une partie de sortie dudit capnomètre au niveau de laquelle l'air sort dudit capnomètre ; et
un dispositif de déviation pour bloquer l'un desdits premier et deuxième chemins d'écoulement d'air dans le capnomètre à la fois, ledit dispositif de déviation comprenant une vanne de déviation qui est commandée par ladite inspiration/expiration dudit air par ledit utilisateur, moyennant quoi, pendant l'inspiration, la vanne de déviation ouvre le premier chemin d'écoulement d'air entre la partie d'inspiration/expiration et la partie d'admission tout en fermant le deuxième chemin d'écoulement d'air entre la partie d'inspiration/expiration et la sortie et moyennant quoi, pendant l'expiration, la vanne de déviation ouvre le deuxième chemin d'écoulement d'air entre la partie d'inspiration/expiration et la partie de sortie, tandis qu'elle ferme le premier chemin d'écoulement d'air entre la partie d'inspiration/expiration et la partie d'admission.

2. Capnomètre selon la revendication 1, dans lequel ledit émetteur (2) est situé à un premier emplacement sur un premier côté de ladite région d'écoulement d'air, dans lequel ledit détecteur (3) est situé à un deuxième emplacement sur un deuxième côté de ladite région d'écoulement d'air, dans lequel ledit premier côté est adjacent audit deuxième côté, dans lequel ledit réflecteur (4) est situé à un troisième emplacement sur un troisième côté de ladite région d'écoulement d'air, et dans lequel ledit troisième côté est opposé audit premier côté ou opposé audit deuxième côté.

3. Capnomètre selon n'importe quelle revendication précédente, comprenant en outre un tube respiratoire (1), dans lequel ledit tube respiratoire définit un canal entre ledit émetteur/détecteur (2,3) et ledit réflecteur (4) pour ladite région d'écoulement d'air, de préférence dans lequel ledit tube respiratoire peut être retiré dudit capnomètre.

4. Capnomètre selon la revendication 3, dans lequel ledit émetteur (2), ledit détecteur (3) et ledit réflecteur (4) sont montés dans ledit tube respiratoire (1)

5. Capnomètre selon la revendication 4, comprenant en outre une couche optique (5) entre ledit émetteur (2) et ledit réflecteur (4) et/ou entre ledit détecteur (3) et ledit réflecteur (4) pour améliorer une efficacité de collecte de ladite lumière IR émise par ledit émetteur sur ledit détecteur, de préférence dans lequel ladite couche optique comprend du silicium et/ou dans laquelle ladite couche optique comprend un réchauffeur.

6. Capnomètre selon la revendication 3, dans lequel ledit émetteur (2) et ledit détecteur (3) sont situés à l'extérieur dudit tube respiratoire (1), et dans lequel ledit tube respiratoire comprend une partie transmissible dans l'infrarouge moyen (10), ladite partie transmissible dans l'infrarouge moyen étant alignée avec ledit émetteur et ledit détecteur pour permettre à la lumière dans l'infrarouge moyen de passer à travers elle et à travers ledit tube respiratoire (1), de préférence dans lequel un réchauffeur est prévu pour chauffer ladite partie transmissive dans l'infrarouge moyen.

7. Capnomètre selon la revendication 6, dans lequel ledit réflecteur (4) est situé à l'extérieur dudit tube respiratoire (1), et dans lequel ledit tube respiratoire comprend une deuxième partie transmissible dans l'infrarouge moyen (10), ladite deuxième partie transmissible dans l'infrarouge moyen étant alignée avec ledit réflecteur pour permettre à la lumière dans l'infrarouge moyen de passer à travers elle et à travers ledit tube respiratoire.

8. Capnomètre selon n'importe quelle revendication précédente, dans lequel ledit émetteur (2) comprend un émetteur à semi-conducteur dans l'infrarouge moyen III-V et/ou dans lequel ledit détecteur (3) comprend un détecteur à semi-conducteur dans l'infrarouge moyen III-V.

9. Capnomètre selon n'importe quelle revendication précédente, dans lequel ladite région d'écoulement d'air présente une section transversale qui est réduite là où ladite lumière IR traverse ladite région d'écoulement d'air.

10. Capnomètre selon n'importe quelle revendication précédente en combinaison avec un dispositif médical, ledit capnomètre comprenant en outre un moyen de blocage pour empêcher l'air entourant ledit capnomètre de s'écouler dans ledit dispositif médical.

11. Inhalateur comprenant un capnomètre selon l'une quelconque des revendications précédentes, dans lequel ledit capnomètre est configuré pour surveiller un niveau de CO₂ dans l'air inspiré ou expiré à travers ledit inhalateur.

12. Procédé d'étalonnage d'un capnomètre, comprenant :
la fourniture d'un capnomètre selon l'une quelconque des revendications 1 à 10 ;
la mesure, à l'aide dudit capnomètre, d'un niveau de CO₂ dans l'air entourant ledit capnomètre à deux instants avant et après un cycle d'expiration-inspiration d'un utilisateur dudit capnomètre, afin de fournir une première et une seconde entrée de niveau de fond de CO₂ ; et
l'étalonnage dudit capnomètre en fonction d'une différence entre lesdites première et seconde entrées de niveau de fond de CO₂.

13. Procédé selon la revendication 12, dans lequel ledit niveau de CO₂ dans l'air entourant ledit capnomètre est mesuré avant et après une pluralité desdits cycles d'expiration-inspiration.

14. Procédé selon la revendication 12 ou 13, dans lequel l'air expiré par ledit utilisateur sort dudit capnomètre par une première partie du capnomètre qui est différente d'une seconde partie du capnomètre par laquelle l'air inspiré par ledit utilisateur pénètre dans ledit capnomètre.

15. Système comprenant:
un capnomètre selon l'une quelconque des revendications 1 à 10, le capnomètre étant configuré pour délivrer des données de concentration de gaz à partir d'un gaz inspiré/expiré par un patient ; et
un processeur de données configuré pour recevoir lesdites données de concentration de gaz, et configuré pour réaliser le procédé selon les revendications 12 à 14.
